# EUROPÄISCHE PATENTSCHRIFT

(11) **EP 3 050 869 B1**
(45) Veröffentlichungstag und Bekanntmachung des Hinweises auf die Patenterteilung: **02.10.2019**
(21) Anmeldenummer: 15153282.7
(22) Anmeldetag: 30.01.2015
(51) Int. Cl.: C07C 67/29, C07C 69/14

(54) **Verfahren zur Herstellung von substituierten Alkylcycloalkanonen**
Method for the preparation of substituted alkyl cycloalkanones
Procédé de fabrication d'alkylcycloalcanones substituées

(43) Veröffentlichungstag der Anmeldung: 03.08.2016
(73) Patentinhaber: Symrise AG, 37603 Holzminden (DE)
(72) Erfinder: Köckritz, Angela, 12437 Berlin (DE); Atia, Hanan, 18059 Rostock (DE); Eckelt, Reinhard, 18059 Rostock (DE); Panten, Joahnnes, 37671 Hoexter (DE); Koch, Oskar, 37079 Göttingen (DE); Esser, Peter, 37639 Bevern (DE)
(74) Vertreter: Global IP Europe Patentanwaltskanzlei

(56) Entgegenhaltungen:
- DE-A1- 19 853 862
- US-A- 3 856 815
- US-A- 4 268 445

## Beschreibung

### Gebiet der Erfindung

Die vorliegende Erfindung betrifft das Gebiet der Herstellung von substituierten Alkylcycloalkanonen der Formel I

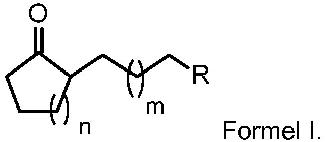

### Stand der Technik

Substituierte Alkylcycloalkanone der Formel I sind wichtige Intermediate, insbesondere für die Synthese von Moschusdüften. Moschusdüfte stellen eine wichtige Komponente von Parfüms dar.

Die Herstellung von ausgewählten Verbindungen der Formel I erfolgt nach DE19853862 A1 über die radikalische Addition von Hydroxy- bzw. Acyloxyalkenen an cyclische Ketone, unter Verwendung eines geeigneten organischen Radikalketteninitiators. Viele der verwendeten Radikalketteninitiatoren weisen beim Gebrauch Nachteile auf. Sie müssen beispielsweise kontinuierlich dem Reaktor zugeführt werden, werden bei der Reaktion irreversibel verbraucht und fördern die Bildung von Reaktionsnebenprodukten, insbesondere von Polymeren. Die Bildung von Polymeren ist besonders bei kontinuierlichen Verfahren nachteilig, da diese über sehr lange Zeiträume ausgeführt werden, so dass sich selbst eine geringe Polymerbildungsgeschwindigkeit nachteilig auswirkt. Die Reaktion muss aufgrund der zunehmenden Viskosität oder wegen zunehmender Ablagerungen von Zeit zu Zeit unterbrochen werden. Zudem besitzen viele der ausdrücklich genannten Radikalketteninitiatoren nachteilige Eigenschaften, wie leichte Entflammbarkeit, Bildung explosiver Gemische, reizender oder giftiger Gase oder Zwischenprodukte. Diese Eigenschaften führen dazu, dass der sicherheitstechnische Aufwand bei der Durchführung der Reaktion sehr hoch sein muss. Bei der Reaktion entstehen neben den gewünschten substituierten Monoalkylcycloalkanonen auch hohe Anteile an substituierten Di-, Tri- und Tetraalkylcycloalkanonen, wodurch die Ausgangsprodukte verstärkt verbraucht werden und nicht mehr wiedergewonnen werden können. Bei der Durchführung des Verfahrens wird aufgrund der hohen anzuwendenden Temperaturen das Arbeiten unter hohem Druck empfohlen, um den Verlust von niedrigsiedenden Stoffen zu reduzieren. Dies erfordert eine spezielle druckfeste Ausrüstung.

### Aufgabe der Erfindung

Es ist die Aufgabe der vorliegenden Erfindung, substituierte Alkylcycloalkanone der Formel I mit hoher Geschwindigkeit, hoher Ausbeute, hoher Selektivität, geringerem Energieeinsatz, geringerem Rohstoffverbrauch und weniger Nebenprodukten herzustellen oder/ und die Sicherheit des Prozesses zu erhöhen.

### Beschreibung der Erfindung

Die Erfindung betrifft ein Verfahren zur Herstellung eines substituierten Alkylcycloalkanons der Formel I 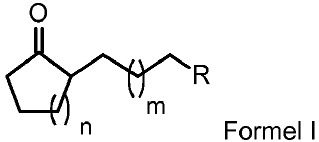 umfassend die Alkylierung eines Cycloalkanons der Formel II 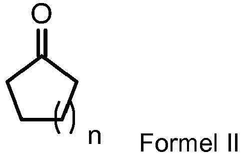 mit einem Alkenderivat der Formel III 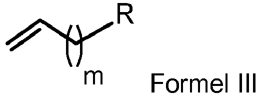 in Gegenwart eines Metalloxids, wobei n 2 bis 20, m 0 bis 10 bedeuten. Verbindungen der Formel I können solche mit n = 3 bis 15 und m = 0 bis 5 sein. R ist eine Hydroxy-, Carboxy-, Carbonyloxyalkyl, Formyloxy-, Alkylcarbonyloxy-, Arylcarbonyloxy, Benzylcarbonyloxyalkylgruppe oder eine der folgenden Gruppen: 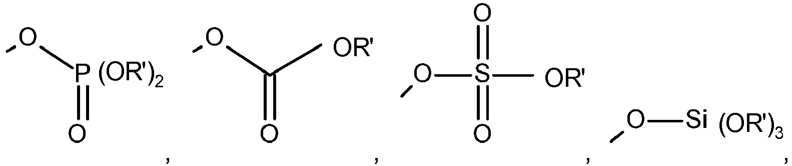 worin R' ein Alkyl-, Aryl-, Heteroaryl-, Alkylaryl- oder Cycloalkylrest ist.

Die Variablen der bevorzugten Verbindungen der Formel II und III sind entsprechend analog.

Überraschend wurde festgestellt, dass bei der Durchführung des erfindungsgemäßen Verfahrens keine Polymere von Verbindungen der Formel II nachgewiesen werden können, so dass das Verfahren kontinuierlich und ohne Unterbrechung über einen sehr langen Zeitraum ausgeführt werden kann. Unter Polymeren werden im Sinne der Erfindung Makromoleküle mit einer Monomeranzahl größer oder gleich 5 verstanden. Außerdem wird der Anteil der gebildeten substituierten Di-, Tri- und Tetraalkylcycloalkanone stark reduziert, wodurch der Verbrauch der Edukte sinkt, folglich mehr Edukte wiedergewonnen und wiedereingesetzt werden können. Auch können die Metalloxide nach erfolgter Reduktion reoxidiert und wiederverwendet werden.

Verbindungen der Formel I sind zum Beispiel 2-(3-Hydroxypropyl)-cyclododecanon und 2-(3-Acetoxypropyl)-cyclododecanon.

Verbindungen der Formel II sind zum Beispiel Cyclohexanon, Cyclooctanon, Cyclododecanon und Cyclohexadecanon.

Verbindungen der Formel III sind zum Beispiel Allylalkohol, Allylacetat, Allylformiat, Allylpropionat, Allylbenzoat und Phenylessigsäureallylester.

Das erfindungsgemäße Verfahren kann sehr gut mit Metalloxiden ausgewählt aus der Gruppe der Kupferoxide, Eisenoxide, Manganoxide, Indiumoxide, Cobaltoxide, Silberoxide und deren Mischungen ausgeführt werden. Besonders gut kann das Verfahren mit Metalloxiden ausgewählt aus Ag₂O, CuO, Fe₂O₃, Fe₃O₄, CuFe₂O₄, Co₃O₄, CoO, MnO₂, In₂O₃ und deren Mischungen ausgeführt werden.

Silberoxide wie Ag₂O bilden unter den Reaktionsbedingungen Silberspiegel oder sehr feinteilige Silberpartikel, die stark in das Reaktionsmedium erodieren, so dass der Aufwand für die Wiedergewinnung, insbesondere die Abtrennung von den restlichen Bestandteilen und die Anlagenreinigung erhöht ist. Bei der Verwendung von Kupferoxiden, Eisenoxiden, Manganoxiden, Indiumoxiden, Cobaltoxiden und deren Mischungen oder/ und CuO, Fe₂O₃, Fe₃O₄, CuFe₂O₄, Co₃O₄, CoO, MnO₂, In₂O₃ und deren Mischungen besteht dieser Nachteil nicht, sie erodieren in dem Verfahren nicht oder nur geringfügig und bilden keine Metallspiegel. Der Aufwand für die Wiedergewinnung und die Anlagenreinigung ist folglich geringer.

Die Metalloxide können bevorzugt als Pulver oder Granulat eingesetzt werden. Die Metalloxide können auch auf einem geeigneten anorganischen Trägermaterial, wie beispielsweise Aluminiumoxid, aufgebracht sein.

Es ist vorteilhaft, wenn das Verfahren bei Temperaturen im Bereich von 100 bis 250 °C abläuft, bevorzugt im Bereich von 150 bis 220 °C und insbesondere bevorzugt im Bereich von 160 bis 190 °C. Das Verfahren kann bei atmosphärischem Druck oder gegebenenfalls unter Überdruck ausgeführt werden.

Im diskontinuierlichen Verfahren kann die Einsatzkonzentration des Metalloxids 1-50 Mol% bezogen auf die Stoffmenge an Cycloalkanon der Formel II und bevorzugt 5-25 Mol% bezogen auf die Stoffmenge an Cycloalkanon der Formel II betragen. Im kontinuierlichen Verfahren ist es zweckmäßiger, die Raumgeschwindigkeit (Quotient aus dem kontinuierlich zugeführten Massenstrom an Verbindungen der Formeln II und III und der Masse des Metalloxids) anzugeben. Sie beträgt bevorzugt 0,01 bis 1 pro Stunde und besonders bevorzugt 0,02 bis 0,5 pro Stunde.

Das molare Verhältnis von Cycloalkanon der Formel II zu Alkenderivat der Formel III kann zwischen 1:1 und 10:1 und bevorzugt zwischen 2:1 und 8:1 liegen.

Das Verfahren kann kontinuierlich oder diskontinuierlich ausgeführt werden. Für die Durchführung des diskontinuierlichen Verfahrens eignet sich beispielsweise ein diskontinuierlicher Rührkesselreaktor. Für das kontinuierliche Verfahren eignet sich beispielsweise ein kontinuierlicher Rohrreaktor, Rührkesselreaktor, Festbettreaktor oder Rieselbettreaktor.

Nach der Durchführung des Verfahrens liegt eine Produktmischung vor, die je nach der Dauer und der angewendeten Verfahrensparameter ein Gemisch mit variierender Konzentration aus den Edukten und Produkten darstellt. Die Produktmischung lässt sich durch geeignete Trennverfahren, insbesondere durch Destillation auftrennen, wodurch die bereits hohe Reinheit der gewünschten alpha-Monoalkylprodukte weiter gesteigert werden kann und gegebenenfalls nicht umgesetzte Edukte wiedergewonnen und wiedereingesetzt werden können.

Das eingesetzte Metalloxid wird durch die Reaktion reduziert. Beim reduzierten Metalloxid handelt es sich um das aus dem Metalloxid gebildete Metall, Metalloxid einer geringeren Oxidationsstufe oder Mischungen daraus. In dem Verfahren bildet sich das reduzierte Metalloxid an der Oberfläche des festen Metalloxids.

Ein weiterer Vorteil der Erfindung ist es, dass das Metalloxid regeneriert (reoxidiert) werden kann, nachdem es durch die erfindungsgemäße Reaktion reduziert worden ist. Die Reoxidation kann durch den Kontakt des reduzierten Metalloxids mit einem sauerstoffhaltigen Gas erfolgen. Die Reoxidation des reduzierten Metalloxids kann besonders effektiv mit Hilfe eines erhitzten sauerstoffhaltigen Gasstroms erfolgen, wobei der Gasstrom eine Temperatur von 100 bis 500°C hat. Der Sauerstoffgehalt des Gases kann mindestens 0,1 Vol.-% Sauerstoff, bezogen auf die Gesamtmenge sauerstoffhaltigen Gases, bestimmt bei 20°C und 1013,25 hPa enthalten, um die Reoxidation in kurzer Zeit ablaufen zu lassen.

Das reduzierte Metalloxid kann vor dem Kontakt mit dem sauerstoffhaltigen Gas von der Produktmischung separiert werden, um es zu reoxidieren. Bei dieser Vorgehensweise beträgt der bevorzugte Sauerstoffgehalt des sauerstoffhaltigen Gases mindestens 17 Vol.-% und dessen Temperatur zwischen 150 und 500°C, um eine besonders schnelle Reoxidation zu ermöglichen. Das auf diese Weise regenerierte Metalloxid zeigt beim Wiedereinsatz in der erfindungsgemäßen Reaktion die gleiche Aktivität wie das ursprünglich eingesetzte Metalloxid. Diese Vorgehensweise kann beispielsweise in einem diskontinuierlichen Verfahren angewendet werden. Es eignet sich auch für ein kontinuierliches Verfahren, wobei beispielsweise zu Beginn des Prozesses mindestens zwei voneinander getrennte Teilmengen von Metalloxid vorliegen. Zunächst wird eine der Teilmengen des Metalloxids mit einem Eduktstrom beschickt und ein Produktstrom abgeführt. Nach einer teilweisen oder vollständigen Reduktion des Metalloxids wird der Eduktstrom auf eine andere Teilmenge des Metalloxids geleitet und die Reaktion darüber betrieben. Währenddessen wird die erste Teilmenge, die nun zu einem gewissen Grad reduziert vorliegt, von der Produktmischung separiert und mit dem sauerstoffhaltigen Gasstrom reoxidiert. Anschließend kann auf die erste oder eine weitere Teilmenge Metalloxid umgeschaltet werden und der Prozess somit kontinuierlich betrieben werden.

Besonders vorteilhaft lässt sich das Verfahren betreiben, wenn während des Ablaufs der Alkylierungsreaktion ein sauerstoffhaltiger Gasstrom in den Reaktor eingeleitet wird. Dabei sollte der sauerstoffhaltige Gasstrom mit dem reduzierten Metalloxid in Kontakt kommen. Das Verfahren kann so, bei entsprechender kontinuierlicher Zufuhr der Edukte und Abführung der Produktmischung, kontinuierlich mit geringem Aufwand über einen sehr langen Zeitraum betrieben werden. Oxidative und reduktive Vorgänge laufen gleichzeitig ab. Der bevorzugte Sauerstoffgehalt des sauerstoffhaltigen Gases beträgt bei dieser Verfahrensführung 0,1 bis 20 Vol.-% und besonders bevorzugt 3 bis 16 Vol.-% damit besonders wenige Nebenprodukte aus radikalischen Nebenreaktionen entstehen und eine schnelle Reoxidation des reduzierten Metalloxids ermöglicht wird. Die Temperatur des sauerstoffhaltigen Gasstroms sollte dabei identisch zur oder nahe bei der Temperatur im Reaktor sein.

### Beispiel 1:

Ein Rührkesselreaktor wird mit 45,5 g Cyclododecanon (CDD) und 5,01 g Allylacetat (AlAc) und Kupferoxid (CuO) (Molverhältnis CDD:AlAc 3:1) befüllt und für 24 h bei 160°C gerührt. Anschließend wurde das Kupferoxid (CuO) abfiltriert und das Reaktionsgemisch gaschromatografisch analysiert und die Selektivität des Verfahrens mit der Formel S = (Stoffmenge gebildetes alpha-Monoalkylprodukt) / (Stoffmenge eingesetztes CDD) berechnet. Sie beträgt 70%.

Im Beispiel 1 von DE 19853862 (Vergleichsbeispiel aus dem bisherigen Stand der Technik) beträgt die Selektivität lediglich 12%.

Im erfindungsgemäßen Verfahren wird folglich bezogen auf das eingesetzte CDD wesentlich mehr des gewünschten alpha-Monoalkylprodukts gebildet.

### Beispiel 2:

Ein Festbettglasreaktor mit einem Durchmesser von 1,9 cm, ausgestattet am Reaktorkopf mit einer Dosierungseinrichtung für Flüssigkeiten und Gase sowie einer Doppelmantelheizung und einer Vorrichtung zum Sammeln des Produktgemisches am Reaktorausgang, wird mit der vorgesehenen Menge an granuliertem CuO (50,7 g; 17,5 ml; Partikeldurchmesser 0,71-1,25 mm) gefüllt. Das Granulat wird aus einem pulvrigen CuO durch Pressen, Zerkleinern der Pellets und Sieben der gewünschten Fraktion erhalten. Oberhalb und unterhalb des Initiatorgranulats wird das freie Volumen des Reaktorinnenraums zur Minimierung von Totvolumen und Fixierung des Initiators mit inerten Korundpartikeln ausgefüllt. Das Gemisch von Cyclododecanon (CDD) und Allylacetat (AlAc) lässt man im Verhältnis 1:7,5 mit einer Flussrate von 2,9 g/h über das auf 180 °C beheizte Initiatorbett strömen. Gleichzeitig wird Luft durch das Reaktorbett mit einer Geschwindigkeit von 2 ml/min geleitet. Am Ausgang des Reaktors wird das Produktgemisch aufgefangen und mittels gaschromatografischer Analyse wird seine Zusammensetzung ermittelt. Die Selektivität zum monoalkylierten Produkt beträgt 75,3%. Unumgesetztes CDD wird durch Destillation von den Produkten abgetrennt und zurückgeführt.

## Patentansprüche

1. Verfahren zur Herstellung eines substituierten Alkylcycloalkanons der Formel I 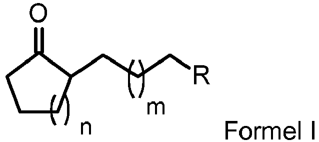 umfassend die Alkylierung eines Cycloalkanons der Formel II 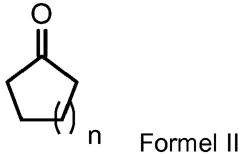 mit einem Alkenderivat der Formel III 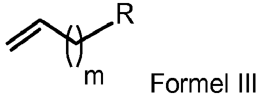 in Gegenwart eines Metalloxids, wobei n 2 bis 20, m 0 bis 10 bedeuten und R eine Hydroxy-, Carboxy-, Carbonyloxyalkyl, Formyloxy-, Alkylcarbonyloxy-, Arylcarbonyloxy-, Benzylcarbonyloxyalkylgruppe oder 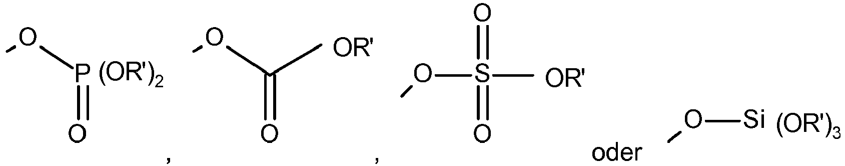 ist, worin R' ein Alkyl-, Aryl-, Heteroary-I, Alkylaryl- oder Cycloalkylrest ist.

2. Verfahren nach Anspruch 1, wobei n 3 bis 15 und m 0 bis 5 bedeuten.

3. Verfahren nach einem der vorherigen Ansprüche, wobei das Metalloxid ausgewählt ist aus der Gruppe der Kupferoxide, Eisenoxide, Manganoxide, Indiumoxide, Cobaltoxide, Silberoxide und deren Mischungen.

4. Verfahren nach einem der vorherigen Ansprüche, wobei das Metalloxid ausgewählt ist aus Ag₂O, CuO, Fe₂O₃, Fe₃O₄, CuFe₂O₄, Co₃O₄, CoO, MnO₂, In₂O₃ und deren Mischungen.

5. Verfahren nach einem der vorherigen Ansprüche, wobei das Metalloxid ausgewählt ist aus der Gruppe der Kupferoxide, Eisenoxide, Manganoxide, Indiumoxide, Cobaltoxide und deren Mischungen.

6. Verfahren nach einem der vorherigen Ansprüche, wobei die Metalloxide ausgewählt sind aus CuO, Fe₂O₃, Fe₃O₄, CuFe₂O₄, Co₃O₄, CoO, MnO₂, In₂O₃ und deren Mischungen.

7. Verfahren nach einem der vorherigen Ansprüche, wobei die Alkylierung bei einer Temperatur im Bereich von 100 bis 250 °C erfolgt, bevorzugt im Bereich von 150 bis 220 °C und insbesondere bevorzugt im Bereich von 160 bis 190 °C.

8. Verfahren nach einem der vorherigen Ansprüche, wobei das molare Verhältnis von Cycloalkanon der Formel II zu Alkenderivat der Formel III zwischen 1:1 bis 10:1 und bevorzugt zwischen 2:1 und 8:1 liegt.

9. Verfahren nach einem der vorherigen Ansprüche, wobei das reduzierte Metalloxid durch ein sauerstoffhaltiges Gas reoxidiert wird.

10. Verfahren nach Anspruch 9, wobei das sauerstoffhaltige Gas mindestens 0,1 Vol.-% Sauerstoff, bezogen auf das Gesamtvolumen sauerstoffhaltigen Gases, bestimmt bei 20°C und 1013,25 hPa, enthält.

11. Verfahren nach einem der Ansprüche 9 oder 10, umfassend das Einleiten eines sauerstoffhaltigen Gasstroms in den Reaktor während des Ablaufs der Alkylierungsreaktion.

12. Kontinuierliches oder diskontinuierliches Verfahren nach einem der vorherigen Ansprüche.

## Claims

1. Process for producing a substituted alkyl cycloalkanone of formula I 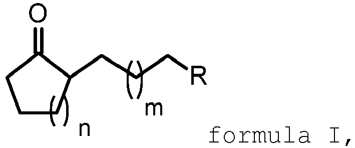 comprising the alkylation of a cycloalkanone of formula II 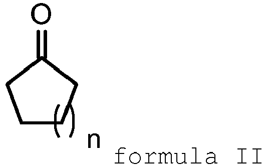 with an alkene derivative of formula III 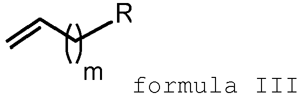 in the presence of a metal oxide, wherein n is 2 to 20, m is 0 to 10, and R is a hydroxyl, carboxyl, carbonyloxyalkyl, formyloxy, alkylcarbonyloxy, arylcarbonyloxy or benzylcarbonyloxyalkyl group or 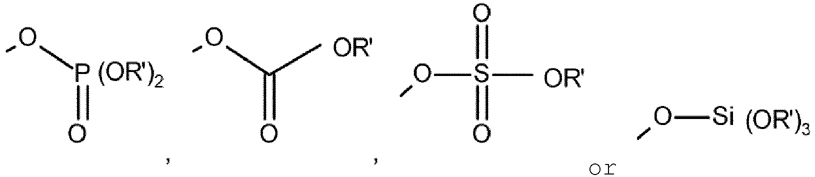 in which R' is an alkyl, aryl, heteroaryl, alkylaryl or cycloalkyl radical.

2. Process according to claim 1, wherein n is 3 to 15 and m is 0 to 5.

3. Process according to one of the preceding claims, wherein the metal oxide is selected from the group consisting of copper oxides, iron oxides, manganese oxides, indium oxides, cobalt oxides, silver oxides, and mixtures thereof.

4. Process according to one of the preceding claims, wherein the metal oxide is selected from Ag₂O, CuO, Fe₂O₃, Fe₃O₄, CuFe₂O₄, Co₃O₄, CoO, MnO₂, In₂O₃, and mixtures thereof.

5. Process according to one of the preceding claims, wherein the metal oxide is selected from the group consisting of copper oxides, iron oxides, manganese oxides, indium oxides, cobalt oxides, and mixtures thereof.

6. Process according to one of the preceding claims, wherein the metal oxides are selected from CuO, Fe₂O₃, Fe₃O₄, CuFe₂O₄, Co₃O₄, CoO, MnO₂, In₂O₃, and mixtures thereof.

7. Process according to one of the preceding claims, wherein the alkylation takes place at a temperature in the range from 100 to 250°C, preferably in the range from 150 to 220°C and particularly preferably in the range from 160 to 190°C.

8. Process according to one of the preceding claims, wherein the molar ratio of cycloalkanone of formula II to alkene derivative of formula III is between 1:1 to 10:1 and preferably between 2:1 and 8:1.

9. Process according to one of the preceding claims, wherein the reduced metal oxide is reoxidized by an oxygen-containing gas.

10. Process according to claim 9, wherein the oxygen-containing gas contains at least 0.1 vol% oxygen, based on the total volume of oxygen-containing gas, determined at 20°C and 1013.25 hPa.

11. Process according to one of claims 9 or 10, which comprises introducing an oxygen-containing gas stream into the reactor during the course of the alkylation reaction.

12. Continuous or batchwise process according to one of the preceding claims.

## Revendications

1. Procédé de production d'une alkylcycloalcanone substituée de formule I 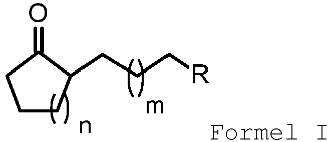 comprenant l'alkylation d'une cycloalcanone de formule II 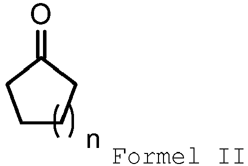 avec un dérivé d'alcène de formule III 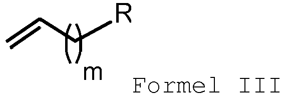 en présence d'un oxyde métallique, où n est un nombre de 2 à 20, m est un nombre de 0 à 10 et R est un groupe hydroxyle, carboxyle, carbonyloxyalkyle, formyloxyle, alkylcarbonyloxyle, arylcarbonyloxyle, benzylcarbonyloxyalkyle ou 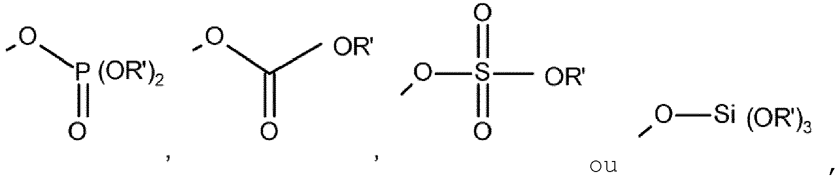 R' étant un reste alkyle, aryle, hétéroaryle-I, alkylaryle ou cycloalkyle.

2. Procédé selon la revendication 1, où n est un nombre de 3 à 15 et m est un nombre de 0 à 5.

3. Procédé selon l'une des revendications précédentes, où l'oxyde métallique est sélectionné dans le groupe des oxydes de cuivre, oxydes de fer, oxydes de manganèse, oxydes d'indium, oxydes de cobalt, oxydes d'argent et leurs mélanges.

4. Procédé selon l'une des revendications précédentes, où l'oxyde métallique est sélectionné entre Ag₂O, CuO, Fe₂O₃, Fe₃O₄, CuFe₂O₄, Co₃O₄, CoO, MnO₂, In₂O₃ et leurs mélanges.

5. Procédé selon l'une des revendications précédentes, où l'oxyde métallique est sélectionné dans le groupe des oxydes de cuivre, oxydes de fer, oxydes de manganèse, oxydes d'indium, oxydes de cobalt et leurs mélanges.

6. Procédé selon l'une des revendications précédentes, où les oxydes métalliques sont sélectionnés entre Fe₂O₃, Fe₃O₄, CuFe₂O₄, Co₃O₄, CoO, MnO₂, In₂O₃ et leurs mélanges.

7. Procédé selon l'une des revendications précédentes, où l'alkylation a lieu à une température comprise entre 100 et 250 °C, avantageusement entre 150 et 220 °C et préférentiellement entre 160 et 190 °C.

8. Procédé selon l'une des revendications précédentes, où le rapport molaire entre cycloalcanone de formule II et dérivé d'alcène de formule III est compris entre 1:1 et 10:1, préférentiellement entre 2:1 et 8:1.

9. Procédé selon l'une des revendications précédentes, où l'oxyde métallique réduit est réoxydé par un gaz à teneur en oxygène.

10. Procédé selon la revendication 9, où le gaz à teneur en oxygène contient au moins 0,1 % en volume d'oxygène relativement au volume total du gaz à teneur en oxygène, déterminé à 20°C et 1013,25 hPa.

11. Procédé selon la revendication 9 ou la revendication 10, comprenant l'application d'un flux de gaz à teneur en oxygène dans le réacteur pendant le déroulement de la réaction d'alkylation.

12. Procédé continu ou discontinu selon l'une des revendications précédentes.
